# EUROPÄISCHE PATENTSCHRIFT

(11) **EP 2 941 211 B1**
(45) Veröffentlichungstag und Bekanntmachung des Hinweises auf die Patenterteilung: **21.09.2016**
(21) Anmeldenummer: 15700248.6
(22) Anmeldetag: 14.01.2015
(51) Int. Cl.: A61B 17/30, A61B 17/29

(54) **CHIRURGISCHER HANDGRIFF FÜR EIN ROHRSCHAFTWERKZEUG**
SURGICAL HANDLE FOR A TOOL HAVING A TUBULAR SHAFT
POIGNÉE CHIRURGICALE POUR UN OUTIL À TIGE TUBULAIRE

(30) Priorität: 21.01.2014 DE 102014100603
(43) Veröffentlichungstag der Anmeldung: 11.11.2015
(73) Patentinhaber: Aesculap AG, 78532 Tuttlingen (DE)
(72) Erfinder: SCHWEITZER, Tom, 78532 Tuttlingen (DE); SAUTER, Wolfgang, 78603 Renquishausen (DE)
(74) Vertreter: Winter, Brandl, Fürniss, Hübner, Röss, Kaiser, Polte - Partnerschaft mbB
(86) Internationale Anmeldenummer: PCT/EP2015/050538
(87) Internationale Veröffentlichungsnummer: WO 2015/110323

(56) Entgegenhaltungen:
- EP-A1- 1 095 641
- WO-A1-2014/202243
- US-A- 6 096 059

## Beschreibung

Die vorliegende Erfindung bezieht sich auf den Bereich chirurgischer Instrumente, insbesondere auf einen axialen Handgriff für chirurgische Rohrschaftinstrumente gemäß dem Oberbegriff des Patentanspruchs 1 und ein chirurgisches Instrument vorzugsweise der minimal invasiven Bauart gemäß dem Oberbegriff des Patentanspruchs 14

In der nachfolgenden Beschreibung werden die Begriffe "proximal" als "nah am Handhabenden" und "distal" als "entfernt vom Handhabenden" benutzt.

### Hintergrund der Erfindung

In der Chirurgie werden Operationen vielfach in einer Art durchgeführt, die "minimal invasiv" genannt wird. Dabei erfolgen Eingriffe innerhalb des Körpers durch kleine Einschnitte und werden mit geeigneten, meist filigranen Instrumenten durchgeführt, die von extern, also von ausserhalb des Patientenkörpers durch den Operateur bedient werden. Für den Patienten bedeutet das eine geringere Belastung während der Operation da keine grossen Schnitte mehr getätigt werden müssen, d.h. die Wunden bleiben sehr klein. Angewendet wird diese Methode beispielsweise in der Herzchirurgie, Laparoskopie (Bauchhöhlenuntersuchung), Arthroskopie. Manipulative Eingriffe wie Gewebeprobenentnahme oder Entfernung von Organen (z.B. Gallenblasenentfernung) sind hiermit möglich.

Zu diesem Zweck werden sogenannte Rohrschaftinstrumente benutzt. Ein solches Rohrschaftinstrument hat in der Regel einen vorzugsweise hohlen Instrumentenschaft, am dessen distalem Ende ein (Rohrschaft-)Werkzeug beispielsweise in Form einer Schere, Fasszange, Nadelhalter oder dergleichen mechanisch betätigbare Aktuatoren angelenkt ist, mit dem vom Operateur am Patienten gearbeitet werden kann. Am proximalen Ende des Instrumentenschafts befindet sich hierfür eine Vorrichtung in Form einer Handhabe, die es ermöglicht, eine Kraft/Moment, die zur Betätigung des Werkzeugs aufgebracht werden muss, auf einen Kraftübertragungszug vorzugsweise innerhalb des Instrumentenschafts einzuleiten, welcher dann die eingeleitete Kraft/Moment auf das Werkzeug für dessen Betätigung überträgt.

Zur Betätigung des Werkzeugs gibt es spezielle Handhaben in Form von Handgriffen, die an die beschriebenen Instrumente gekoppelt werden können oder fest daran montiert sind. An diese Handgriffe sind hohe Anforderungen bezüglich Reinigung, Zerlegbarkeit, Stabilität oder taktiles Verhalten gestellt.

Ein chirurgischer Handgriff für ein chirurgisches Instrument vorstehend genannter Bauart besteht in der Regel aus einem, mit dem Instrumentenschaft verbindbaren/verbundenen Griffgehäuse, das einen Mechanismus zur Kraft-/Momentenübertragung auf den mit dem Rohrschaftwerkzeug wirkverbundenen Kraftübertragungszug aufnimmt, am Griffgehäuse angelenkte Hebel/Handhaben, welche manuell betätigbar sind und mit dem Mechanismus in Wirkeingriff stehen, ggf. einer distalen Anschlussvorrichtung, über die der Handgriff an den Instrumentenschaft und der Kraftübertragungszug an den gehäuseinternen Mechanismus anschließbar sind und einem Getriebe vorzugsweise in Form einer Kniehebeleinrichtung, welches die Hebel/Handhaben mit dem gehäuseinternen Mechanismus für dessen Betätigung koppelt.

Für den Fall, dass die Handhabe als ein Schwenkhebel ausgeführt ist, der am Griffgehäuse schwenkbar angelenkt ist, ist ein Kniehebel an dessen einem Ende an einem Mittenabschnitt des Schwenkhebels angelenkt, wobei sein anderes Ende an einem im Griffgehäuse axial verschiebbar gelagerten Schieber oder Schub-/Zugstab angelenkt ist, der wiederum mit dem Kraftübertragungszug (z.B. eine Schub-/Zugstange Stange oder Bowdenzug innerhalb des Instrumentenschafts) gekoppelt ist.

Wird nunmehr der Schwenkhebel in Richtung hin zum Instrumentengehäuse manuell geschwenkt, erfährt der Schieber über den Kniehebel eine Axialverschiebung zur Betätigung des distalen Instrumentenwerkzeugs. Um den Schwenkhebel wieder in seine Konstruktionslage zurück zu schwenken, ist am Schieber und/oder am Schwenkhebel selbst eine Rückstellfeder angeordnet, welche bei der manuellen Betätigung des Schwenkhebels gespannt wird und bei Loslassen des Schwenkhebels diesen wieder zurückführt.

Eine Möglichkeit um die Rückstellkraft für die Handhaben zu erzeugen ist eine axiale Druckfeder (Schraubenfeder), die im Griffgehäuse eingebaut ist und den Schieber axial in die Ausgangsstellung zurückstellt, wenn die manuell aufgebrachte Betätigungskraft auf die Handhabe verringert wird. Die eingebaute axiale Druckfeder im Griffgehäuse benötigt allerdings Platz. Nebenbei wirkt die Federkraft in Kraftrichtung gesehen erst nach (distal) der Kniehebelübersetzung. Dadurch verringert sich, in Abhängigkeit von der Winkelstellung des Kniehebels und damit der Handhabe, die Wirkung der Federkraft sehr stark. Bei fast geschlossenem Werkzeug fällt dann die Wirkung der Federkraft stark ab und es kann zu einem unerwünschten, weil nicht kontrollierbaren Zuschnappen des Werkzeugs führen.

Auf dem Markt erhältliche Handgriffe mit dem vorstehend beschriebenen konzeptionellen Aufbau weisen daher diverse Einschränkungen auf. Beispielsweise sind die Handgriffe für Rohrschaftinstrumente aufgrund ihrer komplexen Mechanik oft nicht zerlegbar. Daneben ist das taktile Verhalten bei erhältlichen Handgriffen vorstehenden Aufbaus aufgrund der Nachschaltung der Feder nach dem Kniehebelmechanismus nicht optimal oder geht sogar weitestgehend verloren.

Eine zweite Variante ist die Anbringung einer Feder am Griff selbst, nämlich zwischen Griffgehäuse und Handhabe, so, wie es auch beispielsweise in bekannten Scherenkonstruktionen realisiert ist. Die Federkraft wirkt in diesem Fall direkt zwischen dem Griffgehäuse und der Handhabe und damit in Kraftrichtung gesehen vor dem Kniehebelmechanismus. Hierbei muss die Handhabe jedoch eine hohe Steifigkeit besitzen, was ein dementsprechend hohes Gewicht nach sich zieht.

### Stand der Technik

Das Dokument WO 2013/079340 offenbart einen chirurgischen Handgriff, bei dem mittels eines Gelenkelementes in Form eines Kniehebels ein Schieber innerhalb eines Griffgehäuse axial verschoben wird, um einen nachgeschalteten Kraftübertragungszug zu betätigen. Handhaben/Hebel, die mit den Gelenkelementen in Eingriff stehen, werden hierbei zueinandergedrückt und wirken über die Gelenkelemente auf den Schieber. Die Rückstellkraft auf die Handhaben wird über eine axiale Druckfeder erzeugt, die im Griffgehäuse sitzt und auf den Schieber einwirkt. Zusätzlich besitzt der Handgriff axial auf den Schieber wirkende Anschlagelemente zur Begrenzung von dessen Betätigungsweg, die verhindern sollen, dass der Kniehebelmechanismus umschlägt. Dies bedeutet Montageaufwand für die zusätzlichen Teile, höheres Gewicht und mehr Spalten, die eine Reinigung des Gerätes erschweren.

Dokument US 2008/064929 A1 offenbart einen axialen Handgriff für ein Rohrschaftinstrument, bei dem die Rückstellkraft auf die Betätigungshebel über Blattfedern erzeugt wird, die jeweils einen integralen Bestandteil oder Längsabschnitt der Betätigungshebel ausbilden. Die Betätigungshebel sind massiv und daher schwer, was nachteilig in der Handhabung des Instruments während der Operation ist.

'2. r EP-A-1095641 und US-A-6096059 zeigen beide einen chirurgischen Handgriff mit Federelementen, die als Kniehebel fungieren. 2.

Aufgabe der vorliegenden Erfindung ist es, die vorgenannten Nachteile zu vermeiden und einen chirurgischen Handgriff sowie ein chirurgisches Instrument bereitzustellen, der/das eine gute taktile Rückmeldung an den Bediener gibt, die Möglichkeit der einfachen Zerlegbarkeit bietet und sich leicht reinigen lässt.

Diese Aufgabe wird durch einen chirurgischen Handgriff mit den Merkmalen des Patentanspruchs 1 sowie durch ein chirurgisches Instrument mit den Merkmalen des Anspruchs 14 gelöst. Vorteilhafte Ausgestaltungen der Erfindung sind Gegenstand der Unteransprüche.

Der Kern der vorliegenden Erfindung besteht gemäß einem ersten Aspekt in der Bereitstellung eines chirurgischen Handgriffs für ein chirurgisches Instrument, insbesondere ein Schaftinstrument, zur Betätigung eines distal an einem Instrumentenschaft angeordneten Werkzeugs mit einem Griffgehäuse, an dem zumindest ein Hebel/Handhabe relativ dazu bewegbar/schwenkbar angelagert ist, die über einen Kniehebelmechanismus mit einem im Griffgehäuse axial beweglichen Schieber gekoppelt ist, um durch Betätigung des Hebels/Handhabe aus deren Konstruktionslage, entgegen der Rückstellkraft eines Federelements, den Schieber zu bewegen und dadurch das Werkzeug zu betätigen. Erfindungsgemäß ist derKniehebelmechanismus durch ein federndes Element ausgebildet, oder anders ausgedrückt, der Kniehebel bildet gleichzeitig das federnde Element zur Erzeugung einer Rückstellkraft auf den Hebel/Handhaben.

Durch diese Maßnahme kann auf die Anordnung eines zusätzlichen, separaten Federelements zur Rückstellung des Hebels/Handhabe verzichtet werden, was Platz in der Konstruktion einspart und für andere Zwecke verwendet werden kann, eine leichtere Zerlegung des Handgriffs gewährleistet und durch eine Verminderung der Zahl von engen Zwischenräumen eine bessere Reinigung des Handgriffs und damit des Instrumentes gewährleistet.

Günstig ist es, wenn der chirurgische Handgriff einen ersten Hebel/Handhabe umfasst, der um eine quer zur Griffachse ausgerichtete Schwenkachse am Griffgehäuse schwenkbar gelagert ist. Die schwenkbare Lagerung des ersten Hebels/Handhabe erlaubt deren klar definierte und handhabungsfreundliche Bewegung relativ zum Griffgehäuse. Zur Erzielung eines kompakten Aufbaus des Handgriffes ist der erste Hebel/Handhabe unmittelbar am Griffgehäuse schwenkbar gelagert. "Quer zur Griffachse" bedeutet vorliegend in einer Ebene, senkrecht zu der die Griffachse ausgerichtet ist.

Für die Handhabung ist es vorteilhaft, wenn der erste Hebel/Handhabe mit seinem proximalen Ende schwenkbar gelagert ist und mit seinem distalen, freien Ende relativ zum Griffgehäuse schwenkbar ist. Dadurch kann der erste Hebel/Handhabe vom Benutzer auf einfachere Weise ergriffen und betätigt werden.

Vorzugsweise ist der erste Hebel/Handhabe an einem proximalen oder nahe einem proximalen Ende des chirurgische Handgriffs schwenkbar gelagert, denn dies erlaubt es, dem chirurgischen Handgriff eine kompakte Konstruktion zu verleihen.

Bevorzugt ist der erste Hebel/Handhabe ausgehend von einer abgespreizten Stellung relativ zum Griffgehäuse, die er in der nicht betätigten Stellung (Konstruktionslage) einnimmt, in eine angenäherte Stellung relativ zum Griffgehäuse, die er in der mindestens einen Betätigungsstellung einnimmt, und umgekehrt, überführbar. Zum Betätigen des chirurgischen Handgriffs kann der erste Hebel/Handhabe von der abgespreizten Stellung in die angenäherte Stellung unter Verschwenken relativ zum Griffgehäuse überführt werden. Dies erleichtert einem Benutzer die Handhabung des chirurgischen Handgriffs.

Günstig ist es, wenn der erste Hebel/Handhabe in zumindest einer Betätigungsstellung fixierbar ist, denn dies gibt die Möglichkeit, auch den Schieber und damit das Kraftübertragungselement in einer Stellung zu fixieren. Dies ist beispielsweise günstig, wenn am distalen Ende des Rohrschaftwerkzeuges Maulteile zum Fassen von Körpergewebe oder eines chirurgischen Instrumentes wie etwa einer Nadel angeordnet sind.

Die Kraftübertragung auf das Rohrschaftwerkzeug erfolgt im Allgemeinen über die Kniehebelübersetzung. Das übertragende Element zwischen Handhabe und Schieber ist bei einer herkömmlichen Kniehebelübersetzung nicht federnd ausgebildet weshalb die Rückstellung des Hebels/Handhabe daher über ein zusätzliches elastisches Element, bevorzugt eine Feder, erzeugt werden muss. Ziel ist es daher, mit weniger Bauteilen auszukommen und die Nachteile eines starren Kniehebels zu vermeiden.

Die Erfindung kombiniert quasi die Funktion des an sich steifen Kniehebels mit der Eigenschaft des federnden Elements. Anders ausgedrückt vereinigt dieses Element die Eigenschaften des starren Kniehebels und der Feder in einem Bauteil, wodurch im Griffgehäuse zusätzlicher Bauraum frei wird, der anderweitig nutzbar ist, beispielsweise zur Verbesserung der Koppelvorrichtung an das Rohrschaftinstrument. Das spart Herstellungskosten beim Zusammenbau und vereinfacht durch weniger Spalten die Reinigung.

Die Kombination der beiden Funktionen, Kniehebelübersetzung und Rückstellfeder wird erfindungsgemäß durch ein einziges Bauteil, bevorzugt eine Blattfeder übernommen, die den starren Kniehebel gemäß dem Stand der Technik ersetzt.

Eine Blattfeder hat im Allgemeinen eine, im Wesentlichen, lang gestreckte Form mit einem ersten und einem zweiten Ende. An mindestens einem Ende kann die Blattfeder Mittel zur Befestigung aufweisen, mit denen sie an einem Gegenstück vorzugsweise dem Hebel/Handhabe und/oder Schieber befestigt werden kann.

Die Mittel zur Befestigung an einem oder den beiden Enden der Blattfeder können so beschaffen sein, das sie eine, relativ zu dem Gegenstand an dem sie befestigt sind, starre Verbindung oder aber eine relativ zum Gegenstand, an dem sie befestigt sind, bewegliche/schwenkbare Verbindung gewährleisten.

Diese Mittel können beispielsweise Löcher für Schrauben oder Nieten, Verdickungen des Materials an Enden der Blattfeder, Verbreiterungen, zu Ösen umgebogene Enden der Blattfeder oder ähnliche Mittel sein.

Der Gegenstand (Schieber/Handhabe) an dem die Blattfeder befestigt werden kann hält selbst ebenfalls Mittel bereit, die mit den genannten Mitteln an den Enden der Blattfeder korrespondieren und eine Verbindung zu den Befestigungsmitteln an den Enden der Blattfeder unterstützen.

Diese Mittel können beispielsweise Schraubengewinde, Drehachsen, Klemmschlitze, Klemmschlitze mit Sackloch, Nut-/Feder Verbindung und dergleichen Mittel sein, die geeignet sind, das Ende der Blattfeder mit dem Gegenstand zu verbinden.

Des weiteren können, unterstützend oder allein, zu den genannten Mitteln noch Klebe-, Löt- und Schweissverbindungen treten um das Ende der Blattfeder mit einem Gegenstand (Schieber/Handhabe) zu verbinden.

Bevorzugt ist die Blattfeder gerade und entlang einer Längsachse gestreckt. Die Breite der Blattfeder ist größer als die Dicke/Höhe.

Die Dicke der Blattfeder kann, zum Erreichen einer gewünschten Federkennlinie, unabhängig über die Länge der Blattfeder gewählt werden.

Die Breite der Blattfeder ist, zumindest abschnittsweise, homogen.

Die Blattfeder kann Bereiche aufweisen, in denen sich das Verhältnis von Breite zu Dicke der Feder ändert um unterschiedliche Federeigenschaften zu erreichen.

Für den Zusammenbau kann es günstig sein, wenn die Breite der Blattfeder zu einem oder zu beiden Enden hin abnimmt.

Die Blattfeder kann auch eine gekrümmte Form im spannungsfreien Zustand aufweisen, sodass eine (gedachte) Verbindung zwischen den Enden der Blattfeder kürzer ist, als die Länge der Blattfeder selbst (Bogenform).

Der Krümmungsradius der Blattfeder kann abschnittsweise homogen sein. Ebenso kann die Krümmung der Blattfeder mehrere unterschiedliche Krümmungsradien aufweisen (unter Beibehaltung der Bogenform), um beispielsweise einen Schutz gegen Überlast zu erreichen.

Die Handhaben oder auch Hebel oder auch Griffschalen haben eine obere und eine untere Fläche, wobei eine obere Fläche der Handinnenfläche zugewandt ist, eine untere Fläche ist von der Handfläche abgewandt. Jeder Hebel/Handhabe weist Mittel zur Einleitung einer Kraft auf die Blattfeder auf, um die Blattfeder zu verformen, damit diese eine zur Zurückstellung der Handhabe in eine Ausgangslage benötigte Energie speichert.

Diese Mittel können an der, der Handfläche abgewandten Fläche, d.h. an der dem Griffgehäuse zugewandten Fläche der Handhaben angebracht sein. Die Krafteinleitungsmittel können punktförmig oder flächig auf die Blattfeder in deren Mittenabschnitt einwirken, beispielsweise derart, dass eine zunächst vorgekrümmte (bogenförmige) Blattfeder mit zunehmender Betätigung des Hebels/Handhabe in Geradestellung federelastisch deformiert wird, um dann bei Freigeben des Hebels/Handhabe wieder in die gekrümmte Form zurück zu kehren und dabei den Hebel/Handhabe in die Ausgangsstellung (Konstruktionslage) zurück zu schwenken und gleichzeitig den Schieber zurück zu ziehen. Die Krafteinleitungsmittel können beispielsweise die Form eines Vorsprungs am jeweiligen Hebel/Handhabe annehmen, der an einem Mittenabschnitt der Blattfeder (biegeelastischer Kniehebel) anliegt und diesen bei Betätigung des Hebel(Handhabe durchdrückt oder eine Art Kulissenführung sein, entlang der sich die Blattfeder abrollt und sich dabei streckt.

Die Krafteinleitungsmittel können bei unbelasteten Handhaben, also im Ausgangszustand, die Blattfeder ohne Krafteinleitung berühren.

Die erfindungsgemäße Blattfeder kann aus geeigneten elastischen Materialien bestehen wie beispielsweise Federstahl, Kunststoff, faserverstärkter Kunststoff.

Dadurch, dass beim Zusammendrücken der Handhabe eine kontinuierlich steigende Federspannung erzeugt wird, fällt die Notwendigkeit einer zusätzlichen Druckfeder weg. Durch entsprechende Form der Blattfeder kann zusätzlich ein Überlastschutz erreicht werden, der zum Schutz der filigranen Instrumente sehr wünschenswert ist. Beispielsweise kann die Blattfeder an einem Federendabschnitt vorzugsweise im Bereich des Schiebers gegenüber der Bogenform stärker gekrümmt sein (engerer Radius als im übrigen Federabschnitt), sodass sich dieser Federabschnitt mit kleinerem Biegeradius bei Erreichen oder Überschreiten einer bestimmten Axialkraft auf die Blattfeder (gemäß der allgemeinen Kniehebelfunktion) ausbaucht und damit die Axialkraftübertragung vom Hebel/Handhabe auf den Schieber begrenzt.

Der erfindungsgemäße Handgriff umfasst bevorzugt einen zweiten Hebel/Handhabe, um dem Benutzer die Handhabung des Handgriffes zu erleichtern. Es kann vorzugsweise vorgesehen sein, dass der zweite Hebel/Handhabe unbeweglich am Griffgehäuse festgelegt ist, da hierdurch dem Handgriff eine einfachere konstruktive Ausgestaltung verliehen werden kann.

Der zweite Hebel/Handhabe kann auch beweglich sein und beispielsweise am Griffgehäuse, analog zum ersten Hebel/Handhabe, um eine quer zur Griffachse ausgerichtete Schwenkachse schwenkbar gelagert sein. Ferner kann der zweite Hebel/Handhabe mit dem Schieber durch einen Kniehebelin Wirkverbindung stehen, um eine Betätigungskraft (Axialkraft) auf den Schieber zu übertragen. Bevorzugt wird zur Verbindung von zweitem Hebel/Handhabe und Schieber ebenfalls das erfindungsgemäße federnde Element, insbesondere eine Blattfeder benutzt.

Bei einer Umsetzung des Handgriffes in der Praxis erweist es sich als günstig, wenn der erste Hebel/Handhabe und/oder der zweite Hebel/Handhabe schalenförmig ausgestaltet sind und das Griffgehäuse, insbesondere hülsenartig, in Umfangsrichtung der Griffachse zumindest bereichsweise umgeben. Die Hebel/Handhaben sind beispielsweise an zwei einander gegenüberliegenden Seiten der Griffachse angeordnet und können vom Benutzer mit der Handfläche auf einfachere Weise ergriffen und betätigt werden.

Weiter erweist es sich als vorteilhaft, wenn der erste Hebel/Handhabe und der zweite Hebel/Handhabe jeweils als axial erstreckte halbzylindrische oder im Wesentlichen halbzylindrische Schalen ausgestaltet sind, die das Griffgehäuse zwischen sich aufnehmen.

### Beschreibung der Zeichnungen

Die Erfindung wird nachstehend anhand bevorzugter Ausführungsbeispiele unter Bezugnahme auf die begleitenden Zeichnungen näher erläutert.
Fig.1 zeigt modell haft das Zusammenwirken der Einzelteile und die Rückstellung des Hebels/Handhabe beim Kniehebel mit axialer Druckfeder,
Fig. 2 zeigt das Zusammenwirken der Einzelteile und die Rückstellung des Hebels/Handhabe beim Kniehebel mit Rückstellfeder zwischen Griffgehäuse und Hebel/Handhabe,
Fig.3 zeigt modell haft eine Ausführungsform eines erfindungsgemäßen Handgriffs mit einem federnden Kniehebel,

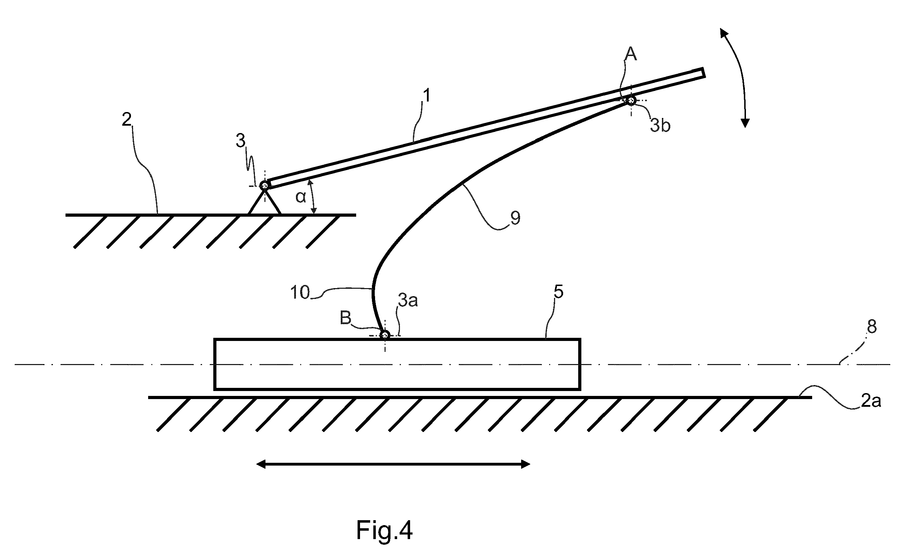

Fig.4 zeigt modell haft eine weitere Ausführungsform eines erfindungsgemäßen Handgriffs mit einem federnden Kniehebel,
Fig. 5a-c zeigen die Funktion des federnden Kniehebels an der Ausführungsform mit gekrümmter Feder,
Fig. 6a-b zeigen die Funktion des federnden Kniehebels an der Ausführungsform mit nicht gekrümmter Feder,
Fig. 7a-d zeigen die verschiedenen Befestigungsmöglichkeiten der Blattfeder am Schieber,
Fig. 8 a-b zeigen die Wirkung der Überlastfunktion,
Fig. 9a-b zeigen die erfindungsgemäße Blattfeder und die Blattfeder mit Überlastfunktion im Detail,
Fig. 10a-c zeigen den realen chirurgischen Handgriff mit erfindungsgemäßer Blattfeder im montierten/Einbauzustand in verschiedenen Ansichten,und
Fig. 11 a-c zeigen den realen chirurgischen Handgriff mit erfindungsgemäßer Blattfeder mit Überlastschutz im montierten/Einbauzustand in verschiedenen Ansichten.

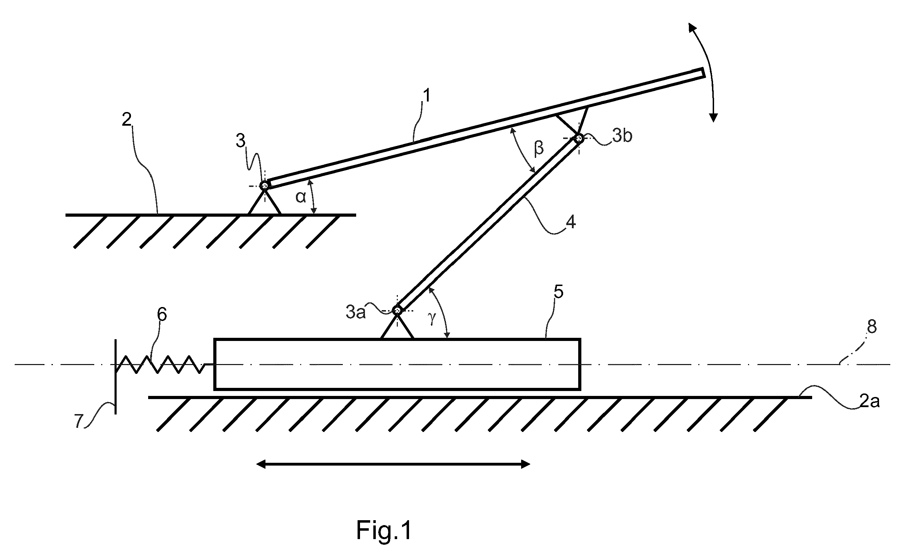

Modellhaft stellt Fig. 1 die Wirkung des federnden Kniehebels in der Ausführung des Standes der Technik dar. Die im realen Handgriff benutzte Handhabe ist hier durch einen einfachen Hebel/Handhabe 1 dargestellt. Der Hebel 1 ist an einem Griffgehäuse, modellhaft dargestellt durch die Bezugszeichen 2, 2a, schwenkbar um eine Achse 3 angelenkt. Vorzugsweise ist die Achse 3 an einem proximalen Ende des Handgriffs angebracht.

An einem distalen Ende des Hebels 1 ist ein Kniehebel 4 ebenfalls um eine Achse 3b schwenkbar gelagert. Weiterhin ist der Kniehebel 4 relativ zu einem Schieber 5 um eine am Schieber 5 angebrachte Achse schwenkbar.

Eine Feder 6 ist in proximaler Position zwischen einem Anschlag 7 im proximalen Teil des Griffgehäuses 2, 2a und dem Schieber 5 der sich innerhalb des Griffgehäuses 2, 2a befindet, montiert.

In der modellhaften Darstellung der Fig. 1 ist die Orientierung der Hebel 1, 4 so, dass sich der zwischen Griffgehäuse 2, 2a und der Handhabe 1 eingeschlossene Winkel α nach proximal und der von Handhabe 1 und Kniehebel 4 eingeschlossene Winkel β in Richtung distal öffnet. Eine Umkehrung der Orientierung und damit eine Umkehrung der Richtung von Ruhelage (Konstruktionslage) und Verschiebung sind damit möglich. Für diesen Fall werden die Feder 6 und der Anschlag 7 spiegelbildlich auf der anderen Seite des Schiebers 5 montiert.

In Fig. 1 wird der aktuelle, nachteilige Stand der Technik anhand der Variante mit axialer Druckfeder als flexibles Rückstellelement erläutert. Das Bezugszeichen 5 bezeichnet dabei den Schieber, der die kräftemäßige Kopplung der über die Handhabe 1 und den Kniehebel 4 auf den Schieber 5 eingeleiteten Kraft auf das Rohrschaftinstrument bewirkt. Das Rohrschaftinstrument selbst, nebst zugehöriger Ankoppelvorrichtung daran ist nicht dargestellt.

Wird der Hebel 1 heruntergedrückt, bewegt sich der damit beweglich verbundene Kniehebel 4 und damit auch der beweglich mit dem Kniehebel 4 verbundene Schieber 5 in dessen Axialrichtung. Der Hebel 4 wird in dieser Anordnung als Kniehebel bezeichnet und ist eine starre, nicht flexible Verbindung.

Je weiter der Hebel 1 um die Achse 3 zum Griffgehäuse 2, 2a geschwenkt wird, umso weiter bewegt der Kniehebel 4 den Schieber 5 gegen die Kraft der Feder 6. Die Feder 6 kann nur solange eine Rückstellkraft auf den Hebel (das Griffteil) 1 aufbringen, solange Hebel 1 bzw. Hebel 4 einen minimalen Winkel zur Griffachse nicht unterschreiten (Kräfteparallelogramm). Es muss immer gewährleistet sein, dass eine nach oben gerichtete Kraft auf den Hebel 1 wirkt. Würde der Kniehebel 4 in eine Position parallel zur Griffachse 8 gelangen, würde es keine nach oben gerichtete Kraft mehr geben und der Kniehebel 4 in seiner Stellung verbleiben. Der chirurgische Handgriff würde in diesem Augenblick sozusagen zuschnappen und damit auch das am distalen Ende des Rohrschaftes angebrachte Werkzeug in Schließposition halten.

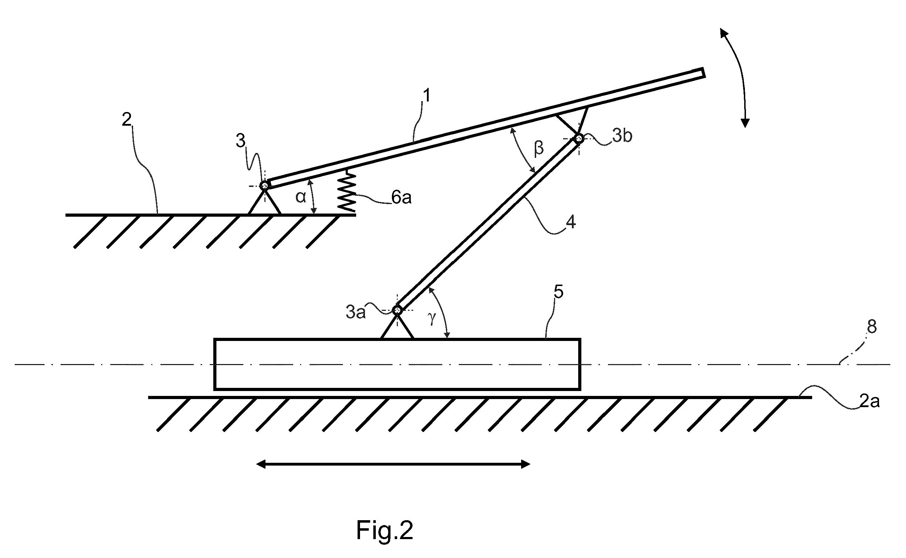

Die zweite, ebenfalls nachteilige Variante des Stands der Technik ist modellhaft in Fig. 2 gezeigt. Die Rückstellfeder 6a ist zwischen Handhabe (Hebel) 1 und dem Griffgehäuse 2, 2a nahe der Schwenkachse 3 angebracht. Das bedingt, dass die Handhabe 1 steif ausgeführt sein muss, was in der Konsequenz bedeutet, dass die Handhabe 1 massiv und schwer ist.

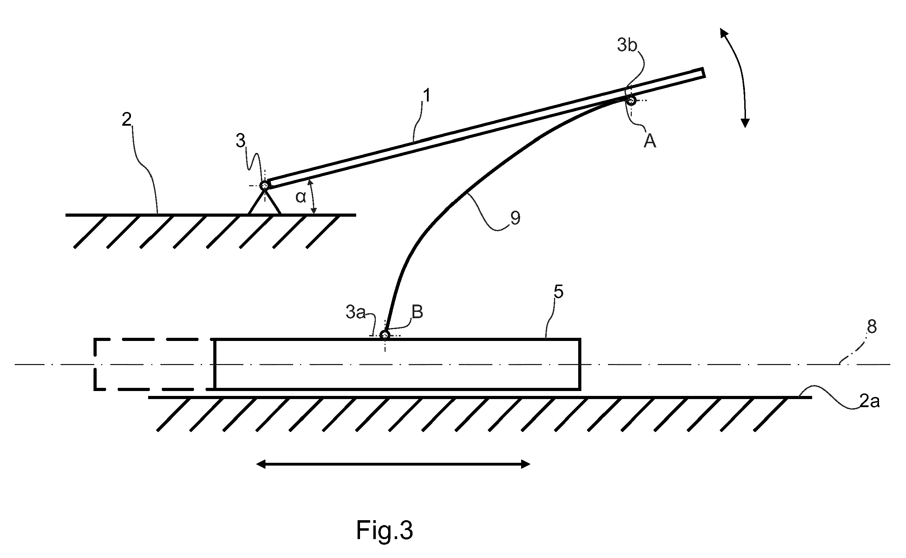

In Fig. 3 sieht man modellhaft das erfindungsgemäße Federelement/Blattfeder 9, welche grundsätzlich den starren Kniehebel sowie die hiervon separat angeordnete Rückstellfeder gemäß dem Stand der Technik ersetzt. Das Federelement/Blattfeder 9 hat ein erstes Ende A und ein zweites Ende B. Mit dem Ende A ist es mit dem Hebel bzw. der Griffschale 1 verbunden, mit dem anderen Ende B ist es mit dem Schieber 5 verbunden.

Das Federelement 9 kann vorzugsweise (bogenförmig) gekrümmt sein. Wird der Hebel/Handhabe 1 um die Achse 3 in Richtung hin zum Griffgehäuse 2, 2a verschwenkt und der zwischen Hebel/Handhabe 1 und Griffgehäuse 2, 2a eingeschlossene Winkel □ verkleinert, vergrößert sich kontinuierlich die Entfernung zwischen den Enden A und B der Blattfeder 9. Das bedeutet für die Blattfeder 9, dass sie aus dem bogenförmigen gekrümmten Zustand heraus, der ihre Ruhelage bedeutet, in Richtung hin zur geraden Ausrichtung ausgelenkt wird und damit Energie für die Rückstellung der Handhaben 1 aufnimmt.

Die Verbindung sowohl zum Schieber 5 als auch zur Handhabe 1 kann als Schwenkachse (3a, 3b) ausgeführt werden; es ist aber auch eine jeweils relativ zur Handhabe 1 als auch zum Schieber 5 starre Verbindung möglich. Eine Kombination beider Befestigungsmöglichkeiten ist auch denkbar.

Die Streckung der Blattfeder 9 während des Verschwenkens des Griffhebels 1 um die Schwenkachse 3 in Richtung hin zum Griffgehäuse wird dadurch bewirkt, dass sich die Blattfeder 9 abschnittsweise an einen dem Griffgehäuse zugewandten Teil der Handhabe 1 anschmiegt (abrollt) und dadurch begradigt wird, also eine Streckung erfährt. Durch die Streckung der Blattfeder 9 sowie durch das Verschwenken des Hebels 1 wird der mit dem Ende der Blattfeder 9 verbundene Schieber 5 entlang der Griffachse 8 axial bewegt.

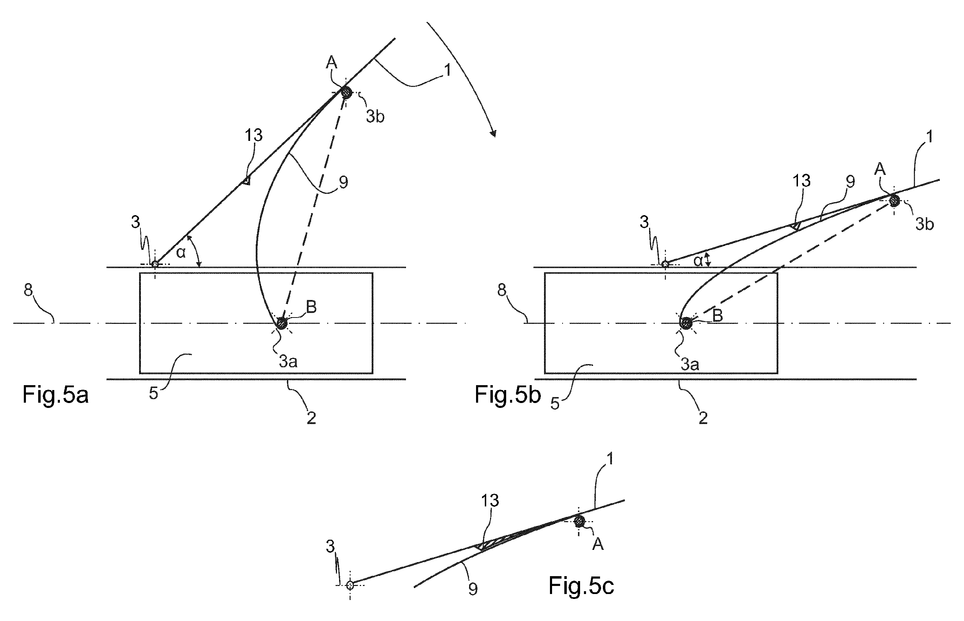

Der untere, dem Griffgehäuse zugewandte Teil der Handhabe 1 kann auch, wie in den Fig. 5a-c gezeigt, Krafteinleitungsmittel 13 (Vorsprung/Zapfen, etc.) beinhalten, die zum Beispiel als vorstehende, Strukturen ausgebildet sein können.

Beim Verschwenken der Handhabe 1 um die Achse 3 kommt das vorstehende Krafteinleitungsmittel 13 mit der Blattfeder 9 in Kontakt und beim weiteren Verschwenken um Achse 3 wird an dieser Stelle die Kraft auf die Blattfeder 9 eingeleitet und verursacht die schon beschriebene Streckung (Fig. 5b). Der Abstand der Krafteinleitungsmittel 13 vom Punkt A und auch deren bauliche Größe bestimmen, wann, bzw. bei welchem Schwenkwinkel der Handhabe 1 relativ zum Griffgehäuse die Streckung der Feder beginnt. Fig. 5c zeigt eine Handhabe 1, bei der das Krafteinleitungsmittel 13 abschnittsweise an der Blattfeder 9 anliegen kann.

Bezüglich der Funktion des federnden Kniehebels 9 gemäß der Fig. 5a bis 5c lässt sich folgendes anführen:

Bei Betätigungsbeginn ist der dargestellte Hebel 1 in Ausschwenkposition und der Schieber 5 entsprechend in zurückgezogener Stellung. Wird ausgehend hiervon der Hebel 1 in Richtung hin zum Griffgehäuse verschwenkt, verschiebt sich der Schieber 5 über den Kniehebel (C-förmige Blattfeder) 9 längs der Achse 8, Dabei wälzt sich die Blattfeder 9 an ihrem Mittenabschnitt kontinuierlich am Vorsprung 13 ab, der die Blattfeder 9 im Mittenabschnitt mit zunehmendem Betätigungsgrad elastisch eindrückt und damit begradigt. Wird der Hebel 1 freigegeben bewirkt die in der Blattfeder 9 gespeicherte Energie, dass sich die Blattfeder 9 sebsttätig in die vorgekrümmte Form zurückbewegt. Dabei wird der Hebel 1 in seine Ausgangsposition zurück geschwenkt und zieht dabei den Schieber 5 in dessen Ursprungsstellung axial zurück.

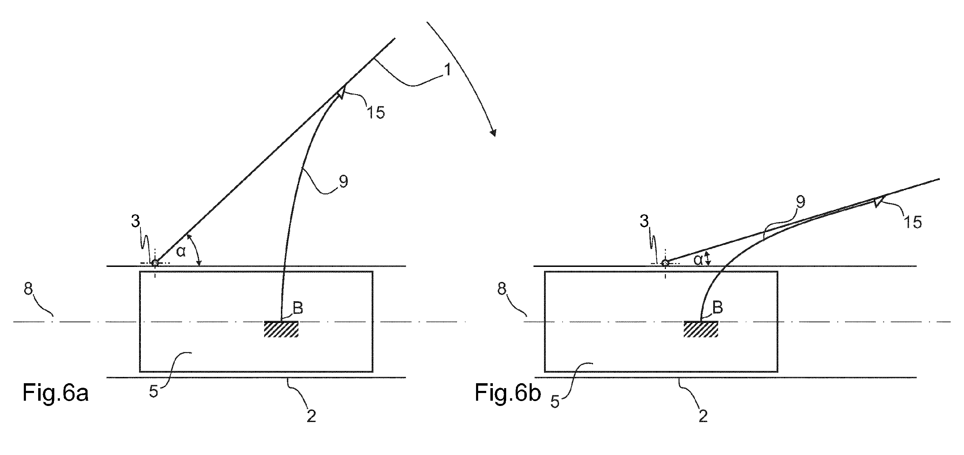

In Fig. 6a, 6b ist eine weitere Variante des federnden Kniehebels gezeigt. In diesem Beispiel ist die Blattfeder 9 starr an den Schieber 5 gekoppelt. Damit entfällt das bewegliche Lager 3 zwischen Blattfeder 9 und Schieber 5.

Der Schieber 5 und die Blattfeder 9 können in diesem Fall als ein komplett zu lieferndes, nicht zerlegbares Teil ausgebildet sein.

In dieser Konstellation kann die Blattfeder 9 vorzugsweise gerade sein. Um die Rückstellwirkung zu erreichen, bewegen sich die Endpunkte A und B der die Blattfeder 9 beim Verschwenken der Handhabe 1 um die Achse 3 nicht voneinander weg, wie in der vorigen Ausführungsform, sondern infolge zunehmender elastischer Biegung der Blatfeder 9 aufeinander zu. Das eine Ende A der Blattfeder 9 kann gegen einen an der, der Handfläche abgewandten, Fläche der Handhabe 1 angebrachten Anschlag 15 stoßen oder die Blattfeder 9 ist am Hebel 1 anscharniert. Der Anschlag 15 (oder Scharnier) verhindert, dass sich das Ende A der Blattfeder 9 beim Zusammenführen der Handhabe 1 relativ zur Handhabe 1 bewegen kann. Der Anschlag 15 (oder Scharnier) stellt somit die Krafteinleitung und damit die Verformung der Blattfeder 9 beim Verschwenken der Handhabe 1 um die Achse 3 sicher. Gleichzeitig wird eine Axialkraft auf die Blattfeder 9 übertragen, wodurch der Schieber 5 längs der Achse 8 verschoben wird.

Fig. 6b zeigt die Blattfeder 9 bei zusammengedrückter Handhabe 1. Die Blattfeder 9 ist jetzt gebogen und kann die Handhabe 1, dadurch, dass sie Verformungsenergie gespeichert hat, wieder in die Ausgangsstellung zurückbringen und gleichzeitig den Schieber 5 in dessen Ursprungsposition zurück ziehen.

Zusätzliche Krafteinleitungsmittel 13 wie in der vorigen Ausführungsform erwähnt, sind nicht nötig, können aber je nach Notwendigkeit, z.B. um eine bestimmte Charakteristik im Kraftverlauf zu erreichen, hinzugefügt werden. Um eine bestimmte Charakteristik der Krafteinleitung zu erreichen, ist es auch möglich, die Blattfeder 9 in einem Winkel relativ zum Schieber 5 anzubringen, wobei die imaginäre Schwenkachse um die dieser Winkel dreht, parallel zur Achse 3 ist. Die Charakteristik der Krafteinleitung ist abhängig vom jeweiligen, fest gewählten Winkel.

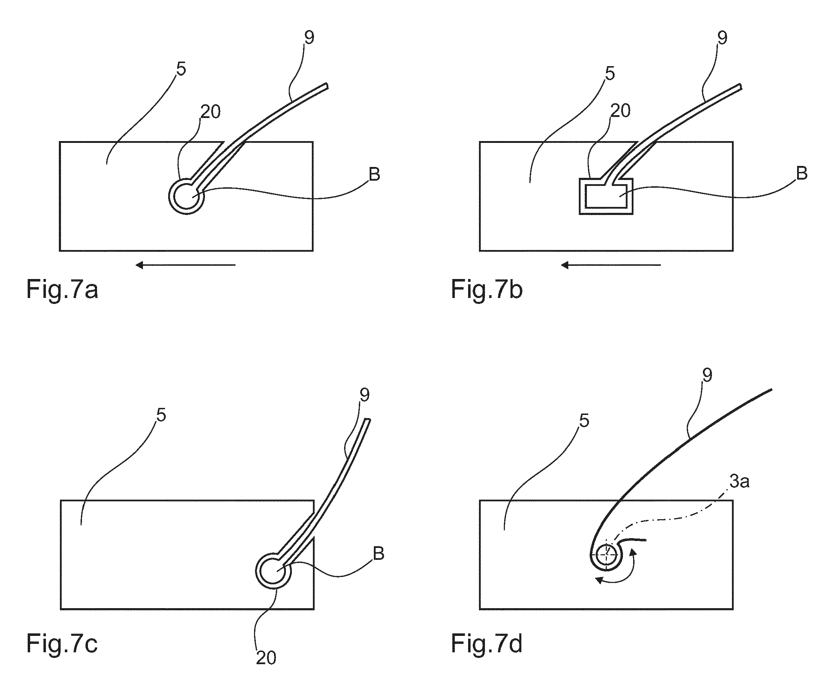

Die Fig. 7a-d illustrieren die verschiedenen möglichen Befestigungsarten (starr und nicht starr) der Blattfeder 9 relativ zum Schieber 5.

Fig. 7a-c zeigen starre Varianten, beispielhaft durch Verbindungen ähnlich dem Nut-Feder System. Am Ende der Blattfeder 9 sind verschiedene Mittel zur Verbindung mit dem Schieber 5. Der Schieber hat dazu die korrespondierenden Mittel.

Denkbar ist auch eine, hier nicht gezeigte Verbindung, die beispielsweise nur geklemmt ist, also ohne dass ein Ende der Blattfeder 9 besondere Befestigungsmittel aufweist. Der Schieber 5 kann im einfachsten Fall einen Schlitz aufweisen in den das eine Ende der Blattfeder geklemmt wird.

Eine Verbindung zwischen Blattfeder 9 und Schieber 5 kann, je nach Material, durch Löten, Kleben, Klemmen erreicht werden

Fig. 7d zeigt eine bewegliche Verbindung, bei dem ein Ende der Blattfeder 9 zu einer Öse gebogen und beweglich um eine Achse 3b am Schieber 5 gelagert ist.

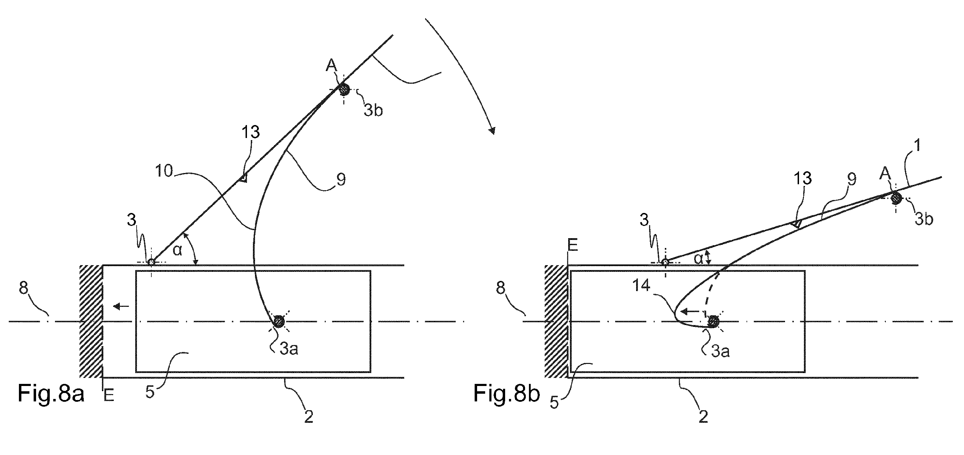

In Fig. 8a-b ist eine weitere Ausführungsform der erfahrungsgemäßen Blattfeder 9 dargestellt. Wie zu sehen, besitzt die Blattfeder 9 eine weitere einfache Krümmung 10 mit einem Krümmungsradius, der kleiner ist als jener der übrigen Bogenform. Diese zusätzliche Krümmung 10, die gut in Fig. 8a (bzw.auch 9b) zu erkennen ist, hat die Aufgabe, das Instrument im Falle einer Überlast zu schützen. Eine Überlast kann dann entstehen, wenn die Handhaben 1 über einen Punkt hinweg geschlossen werden, an dem das Werkzeug an der distalen Seite schon geschlossen ist. Das heißt, bei schon geschlossenen Branchen, beispielsweise einer Zange, können die Handhaben 1 weiter zusammengedrückt werden und es wirkt auf diese eine größer werdende Kraft. Da sich die Blattfeder 9 immer weiter zu einer geraden Form hin verbiegt, also weiter Verformungsenergie speichert je weiter die Handhaben 1 zusammengedrückt werden, wird sie in Richtung Griffachse 8 immer steifer.

Ihre maximale Steifigkeit würde sie erreichen, wenn sie parallel zur Griffachse 8 ausgerichtet wäre. Dieser Extremfall kann zwar nicht eintreten, da die Drehachse 3 der Handhabe1 nicht auf der Griffachse 8 sondern auf dem Griffgehäuse 2, 2a liegt, dennoch kann in diesem Biegezustand die Blattfeder 9 bei weiterem Aufbringen manueller Betätigungskraft auf Hebel/Handhaben 1 soviel Kraft über die Getriebemechanik auf das distale Ende des Rohrschaftwerzeuges übertragen, dass die filigranen Werkzeuge beschädigt werden könnten. Auch eine Beschädigung der Kopplungseinrichtung am proximalen Ende des Rohrschaftwerkzeugs ist möglich.

Abhilfe kann die zusätzliche Krümmung 10 der Blattfeder 9 im gezeigten Bereich bringen. In Fig. 8a wird die Blattfeder 9 in Richtung E bewegt. E repräsentiert hier eine Endstellung, bei der eine weitere Bewegung des Schiebers 5 in Richtung der Griffachse 8 nicht mehr möglich ist. Fig. 8b zeigt was passiert, wenn der Schieber 5 gegen den Endpunkt E stößt.

Wächst die Kraft in Richtung der Griffachse 8 weiter an, siehe Fig. 8b, kommt es neben der Verformung der Blattfeder 9, die die Rückstellkraft aufbaut, zu einer weiteren Verformung / Ausbauchung 14 der Blattfeder 9 im Bereich der zusätzlichen Krümmung 10, was hier zur Illustration übertrieben dargestellt ist. Die überschüssige Kraft ändert somit ihre Richtung und wird in zusätzliche Verformungsenergie gewandelt. Man kann bei der zusätzlichen Krümmung 10 von einem zusätzlichen Hebel bzw. einer zusätzlichen Hebelwirkung auf die Blattfeder 9 sprechen.

Lässt man die Handhabe 1 los bzw. verringert die manuelle Betätigungskraft auf die Hebel/Handhaben 1, bewegt sich zuerst nicht der Schieber 5 zurück, sondern die Blattfeder 9 baut zuerst die zusätzlich gewonnene Energie durch Rückbiegung der Verformung/Ausbauchung 14 in den in Fig.8a gezeigten Zustand ab, bevor sie den Schieber 5 in die Ausgangsstellung zurückfährt und damit auch die Handhaben 1 in ihre Ausgangsstellung bringt.

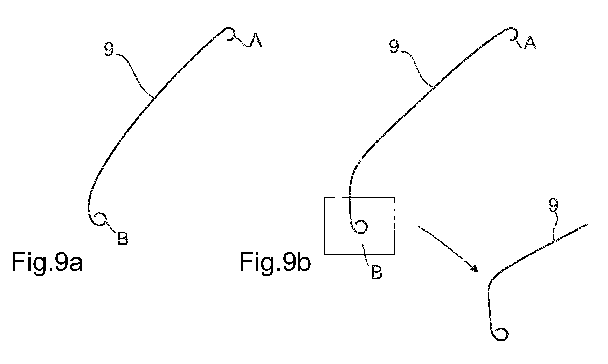

Fig. 9 und 9a zeigen im Detail die erfindungsgemäße Blattfeder 9 ohne (Fig. 9) und mit Überlastkrümmung 10 (Fig. 9a)

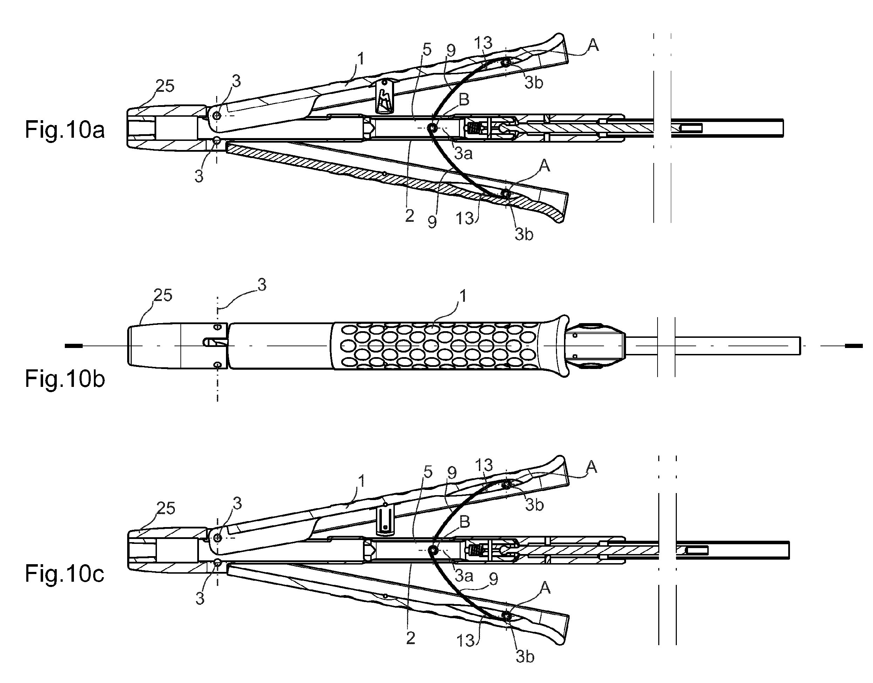

Die Fig. 10a-c zeigen einen konstruktiv realen chirurgischen Handgriff mit erfindungsgemäßer Blattfeder 9 in mehreren Ansichten. Am proximalen Ende des chirurgischen Handgriffs kann ein Luer-Kegel 25 angebracht sein, durch den Spülflüssigkeit geleitet werden kann.

Demzufolge sind die am hier zylindrischen Griffgehäuse 2 anscharnierten Hebel 1 als Hebelschalen ausgeformt, die extrem biegesteif sind. An den zum Griffgehäuse 2 zugewandten Hebelschalen-Seiten sind kulissenförmige Einkerbungen zu sehen, an denen sich bei einer Betätigung der Hebel 1 die C-förmig vorgebogenen Blattfedern 9 in deren Mittenabschnitten abstützen und dadurch federelastisch gerade gedrückt werden. Gleichzeitig wird über die Blattfedern 9 eine Schubkraft auf den Schieber 5 übertragen, der gleitfähig im Griffgehäuse 2 gelagert und geführt ist. Am Schieber 5 ist der Kraftübertragungsmechanismus angekoppelt, der in Fig. 10 als Schub/Zugstange dargestellt ist, die in einem Instrumentenschaft geführt ist.

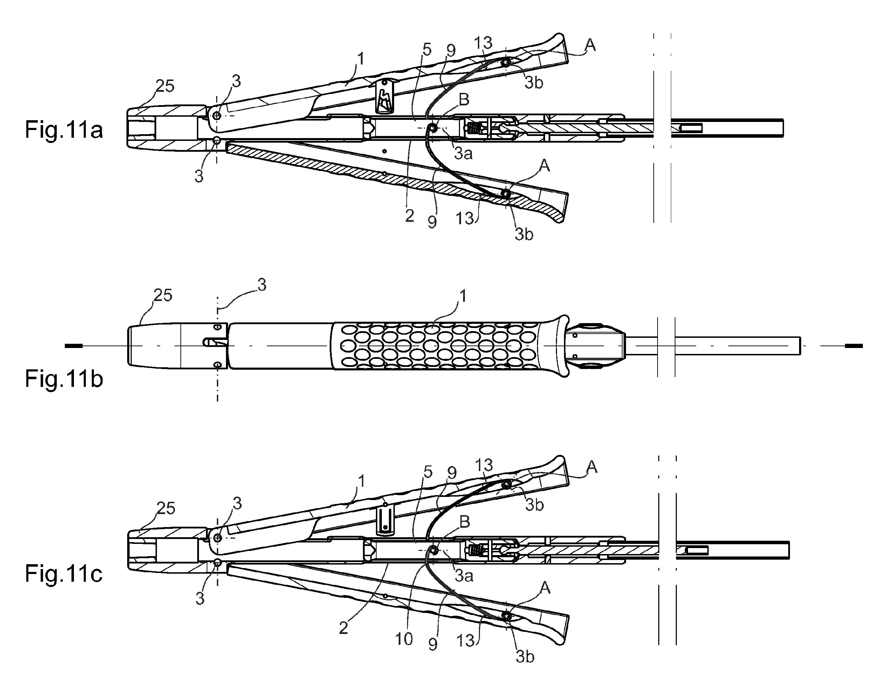

Die Fig. 11a-c zeigen einen chirurgischen Handgriff mit erfindungsgemäßer Blattfeder 9 und zusätzlicher Krümmung 10 als Überlastschutz in mehreren Ansichten. Am proximalen Ende des chirurgischen Handgriffs kann ebenfalls ein Luer-Kegel 25 angebracht sein, durch den Spülflüssigkeit geleitet werden kann. Alle weiteren technischen Merkmale entsprechen dem Handgriff gemäß der Fig. 10.

### Liste der Bezugszeichen

- 1: Hebel, Handhabe
- 2, 2a: Griffgehäuse
- 3, 3a, 3b: Achse
- 4: Kniehebel
- 5: Schieber,
- 6,6a: Feder
- 7: Anschlag
- 8: Griffachse
- 9: federndes Element, Blattfeder
- 10: zusätzlicher Krümmungsradius
- 11: Rohrschaftwerkzeug
- 13: Krafteinleitungsmittel
- 14: Verformung
- 15: mechanischer Anschlag
- 20: Befestigungsmittel
- 25: Luer-Kegel
- A: erster Endpunkt der Blattfeder
- B: zweiter Endpunkt der Blattfeder
- E: Endanschlag
- □: Winkel zwischen Handhabe und Griffgehäuse
- □: Winkel zwischen Handhabe und Kniehebel
- □: Winkel zwischen Kniehebel und Schieber

## Patentansprüche

1. Chirurgischer Handgriff eines chirurgischen Instruments, insbesondere ein Schaftinstrument zur Betätigung eines distal an einem Instrumentenschaft angeordneten Werkzeugs mit einem Griffgehäuse (2, 2a), an dem zumindest ein Hebel/Handhabe (1) relativ dazu bewegbar vorzugsweise schwenkbar angelagert ist, der über einen Kniehebel (4) mit einem im Griffgehäuse (2, 2a) axial beweglichen Schieber (5) gekoppelt ist, um durch Betätigung des Hebels/Handhabe (1) aus dessen Konstruktionslage entgegen der Rückstellkraft eines Federelements den Schieber (5) zu bewegen, wobei das Federelement (9) durch den Kniehebel (4) ausgebildet ist, wobei das eine Federende am Schieber (5) und das andere Federende am Hebel/Handhabe (1) angreift, **dadurch gekennzeichnet, dass** das Federelement (9) an einem Mittenabschnitt zwischen den beiden Federenden am Hebel/Handhabe (1) vorzugsweise an einer am Hebel/Handhabe (1) aus-/angeformten Führungskulisse anliegt, derart, dass bei einer Betätigung des Hebels/Handhabe (1) das Federelement (9) am Hebel/Handhabe (1) abwälzt.

2. Chirurgischer Handgriff eines chirurgischen Instruments nach Anspruch 1 **dadurch gekennzeichnet, dass** der das Federelement (9) ausbildende Kniehebel (4) eine Blattfeder ist.

3. Chirurgischer Handgriff eines chirurgischen Instruments nach Anspruch 1 oder 2 **dadurch gekennzeichnet, dass** das Federelement (9) eine bogenförmige Krümmung in ungespanntem Zustand aufweist.

4. Chirurgischer Handgriff eines chirurgischen Instruments nach einem der vorherigen Ansprüche 2 oder 3 **dadurch gekennzeichnet, dass** der Hebel/Handhabe (1) Mittel zur Krafteinleitung (13) auf die Blattfeder (9) aufweist, um bei einem Betätigen des Hebels/Handhabe (1) ein Durchbiegen der Blattfeder (9) in deren Mittenabschnitt zu bewirken, wobei das Krafteinleitungsmittel (13) vorzugsweise ein am Hebel/Handhabe (1) angeordneter Vorsprung oder weitervorzugsweise eine am Hebel/Handhabe (1) aus-/angeformte Führungskulisse für die Blattfeder (9) ist.

5. Chirurgischer Handgriff eines chirurgischen Instruments nach einem der vorherigen Ansprüche 2 bis 4, **dadurch gekennzeichnet, dass** die Verbindung der Blattfeder (9) relativ zum Schieber (5) scharnierartig oder fest ausgebildet ist.

6. Chirurgischer Handgriff eines chirurgische Instruments nach einem der vorherigen Ansprüche 2 bis 5, **dadurch gekennzeichnet, dass** die Verbindung der Blattfeder (9) relativ zum Schieber (5) unlösbar ausgebildet ist.

7. Chirurgischer Handgriff eines chirurgischen Instruments nach einem der vorhergehenden Ansprüche 2 bis 6, **dadurch gekennzeichnet, dass** die Blattfeder (9) aus Federstahl ist.

8. Chirurgischer Handgriff eines chirurgischen Instruments nach einem der vorhergehenden Ansprüche 2 bis 6, **dadurch gekennzeichnet, dass** die Blattfeder (9) aus einem Kunststoff besteht.

9. Chirurgischer Handgriff eines chirurgischen Instruments nach einem der vorhergehenden Ansprüche 2 bis 8, **dadurch gekennzeichnet, dass** die Blattfeder (9) im Bereich der Verbindung zum Schieber (5) einen weiteren gegenüber dem übrigen Krümmungsradius der Bogenform kleineren Krümmungsradius (10) aufweist, der so gerichtet ist, dass eine Betätigung des zumindest einen Hebels/Handhabe (1) über eine vorbestimmte Schließstellung hinaus eine zusätzliche Verformung oder Ausbauchung der Blattfeder (9) im Bereich des veränderten Krümmungsradius (10) zur Folge hat.

10. Chirurgischer Handgriff eines chirurgischen Instruments nach einem der vorhergehenden Ansprüche, **dadurch gekennzeichnet, dass** der mindestens eine Hebel/Handhabe (1) aus Kunststoff ist.

11. Chirurgischer Handgriff eines chirurgischen Instruments nach einem der vorhergehenden Ansprüche, **dadurch gekennzeichnet, dass** der mindestens eine Hebel/Handhabe (1) mehrteilig ist.

12. Chirurgischer Handgriff eines chirurgischen Instruments nach einem der vorhergehenden Ansprüche, **dadurch gekennzeichnet, dass** der mindestens eine Hebel/Handhabe (1) schalenförmig ausgestaltet ist.

13. Chirurgischer Handgriff eines chirurgischen Instruments nach einem der vorhergehenden Ansprüche **dadurch gekennzeichnet, dass** der mindestens eine Hebel/Handhabe (1) das Griffgehäuse (2, 2a) in Umfangsrichtung der Griffachse (8) zumindest bereichsweise hülsenartig umgibt.

14. Chirurgisches Rohrschaftinstrument bestehend aus einem Rohrschaftwerkzeug, einem Instrumentenschaft, an dessen distalem Ende das Rohrschaftwerkzeug angeordnet ist und einem chirurgischen Handgriff nach einem der vorherigen Ansprüche, der am proximalen Ende des Instrumentschafts angeordnet oder anordenbar ist.

## Claims

1. Surgical handle for a surgical instrument, in particular a shaft instrument for operating a tool arranged distally on an instrument shaft, comprising a handle housing (2, 2a), to which at least one lever/handle (1) is attached so as to be movable, preferably pivotable, relative to said housing, which lever/handle is coupled to a slider (5), which can move axially in the handle housing (2, 2a), by means of a toggle lever (4) in order to move the slider (5) by operating the lever/handle (1) out of its design position, counter to the restoring force of a spring element, the spring element (9) being formed by the toggle lever (4), one end of the spring acting on the slider (5) and the other end of the spring acting on the lever/handle (1), **characterised in that** the spring element (9) abuts the lever/handle (1), preferably a guide link integrally shaped or moulded on the lever/handle (1), at a central portion between the two spring ends such that, when the lever/handle (1) is operated, the spring element (9) rolls on the lever/handle (1).

2. Surgical handle for a surgical instrument according to claim 1, **characterised in that** the toggle lever (4) forming the spring element (9) is a leaf spring.

3. Surgical handle for a surgical instrument according to either claim 1 or claim 2, **characterised in that** the spring element (9) has an arcuate curvature when in the untensioned state.

4. Surgical handle for a surgical instrument according to either preceding claim 2 or preceding claim 3, **characterised in that** the lever/handle (1) comprises means (13) for applying a force to the leaf spring (9) in order to cause the central portion of the leaf spring (9) to bend when the lever/handle (1) is operated, the force-application means (13) preferably being a projection arranged on the lever/handle (1), or more preferably being a guide link for the leaf spring (9) that is integrally shaped or moulded on the lever/handle (1).

5. Surgical handle for a surgical instrument according to any one of preceding claims 2 to 4, **characterised in that** the connection of the leaf spring (9) relative to the slider (5) is hinge-like or rigid.

6. Surgical handle for a surgical instrument according to any one of preceding claims 2 to 5, **characterised in that** the connection of the leaf spring (9) relative to the slider (5) is not releasable.

7. Surgical handle for a surgical instrument according to any one of preceding claims 2 to 6, **characterised in that** the leaf spring (9) is made of spring steel.

8. Surgical handle for a surgical instrument according to any one of preceding claims 2 to 6, **characterised in that** the leaf spring (9) is made of a plastics material.

9. Surgical handle for a surgical instrument according to any one of preceding claims 2 to 8, **characterised in that**, in the region where it is connected to the slider (5), the leaf spring (9) has an additional radius of curvature (10) that is smaller than the other radius of curvature of the arc shape and is designed such that operation of the at least one lever/handle (1) beyond a predetermined closed position results in additional deformation or bulging of the leaf spring (9) in the region of the modified radius of curvature (10).

10. Surgical handle for a surgical instrument according to any one of the preceding claims, **characterised in that** the at least one lever/handle (1) is made of plastics material.

11. Surgical handle for a surgical instrument according to any one of the preceding claims, **characterised in that** the at least one lever/handle (1) is made up of several parts.

12. Surgical handle for a surgical instrument according to any one of the preceding claims, **characterised in that** the at least one lever/handle (1) is shell-shaped.

13. Surgical handle for a surgical instrument according to any one of the preceding claims, **characterised in that** the at least one lever/handle (1) surrounds the handle housing (2, 2a) in the form of a sleeve in the circumferential direction of the handle axis (8) at least in regions.

14. Surgical tube shaft instrument consisting of a tube shaft tool, an instrument shaft, at the distal end of which the tube shaft tool is arranged, and a surgical handle according to any one of the preceding claims that is or can be arranged at the proximal end of the instrument shaft.

## Revendications

1. Poignée chirurgicale d'un instrument chirurgical, en particulier un instrument à tige servant à actionner un outil disposé de manière distale au niveau d'une tige d'instrument, comprenant un boîtier de poignée (2, 2a), au niveau duquel au moins un levier/une manette (1) est monté(e) de préférence de manière à pouvoir pivoter de manière mobile par rapport au boîtier, lequel levier est couplé, par l'intermédiaire d'un levier coudé (4), à un coulisseau (5) axialement mobile dans le boîtier de poignée (2, 2a) afin de déplacer le coulisseau (5) par l'actionnement du levier/de la manette (1) de sa position de construction à l'encontre de la force de rappel d'un élément formant ressort, dans laquelle l'élément formant ressort (9) est réalisé par le levier coudé (4), dans laquelle une extrémité de ressort s'engrène au niveau du coulisseau (5) et que l'autre extrémité de ressort s'engrène au niveau du levier/de la manette (1), **caractérisée en ce que** l'élément formant ressort (9) repose au niveau d'un tronçon central entre les deux extrémités de ressort, au niveau du levier/de la manette (1), de préférence au niveau d'une coulisse de guidage formée au niveau du levier/de la manette (1) de telle manière que dans le cas d'un actionnement du levier/de la manette (1), l'élément formant ressort (9) roule au niveau du levier/de la manette (1).

2. Poignée chirurgicale d'un instrument chirurgical selon la revendication 1, **caractérisée en ce que** le levier coudé (4) réalisant l'élément formant ressort (9) est un ressort à lames.

3. Poignée chirurgicale d'un instrument chirurgical selon la revendication 1 ou 2, **caractérisée en ce que** l'élément formant ressort (9) présente une courbure en forme d'arc dans l'état détendu.

4. Poignée chirurgicale d'un instrument chirurgical selon l'une quelconque des revendications précédentes 2 ou 3, **caractérisée en ce que** le levier/la manette (1) présente des moyens servant à appliquer une force (13) sur le ressort à lames (9) afin de provoquer un fléchissement du ressort à lames (9) dans son tronçon central lors d'un actionnement du levier/de la manette (1),
dans laquelle le moyen d'application de force (13) est de préférence une partie faisant saillie disposée au niveau du levier/de la manette (1) ou mieux encore une coulisse de guidage formée au niveau du levier/de la manette (1) pour le ressort à lames (9).

5. Poignée chirurgicale d'un instrument chirurgical selon l'une quelconque des revendications précédentes 2 à 4, **caractérisée en ce que** la liaison du ressort à lames (9) par rapport au coulisseau (5) est réalisée à la manière d'une charnière ou de manière solidaire.

6. Poignée chirurgicale d'un instrument chirurgical selon l'une quelconque des revendications précédentes 2 à 5, **caractérisée en ce que** la liaison du ressort à lames (9) par rapport au coulisseau (5) est réalisée de manière inamovible.

7. Poignée chirurgicale d'un instrument chirurgical selon l'une quelconque des revendications précédentes 2 à 6, **caractérisée en ce que** le ressort à lames (9) est composé d'un acier ressort.

8. Poignée chirurgicale d'un instrument chirurgical selon l'une quelconque des revendications précédentes 2 à 6, **caractérisée en ce que** le ressort à lames (9) est constitué d'une matière plastique.

9. Poignée chirurgicale d'un instrument chirurgical selon l'une quelconque des revendications précédentes 2 à 8, **caractérisée en ce que** le ressort à lames (9) présente, dans la zone de la liaison par rapport au coulisseau (5), un autre rayon de courbure (10) plus petit par rapport au reste du rayon de courbure de la forme arquée, lequel rayon de courbure est orienté de telle manière qu'un actionnement de l'au moins un levier / de l'au moins une manette (1) a pour conséquence, au-delà d'une position de fermeture prédéterminée, une déformation supplémentaire ou un gauchissement du ressort à lames (9) dans la zone du rayon de courbure (10) modifié.

10. Poignée chirurgicale d'un instrument chirurgical selon l'une quelconque des revendications précédentes, **caractérisée en ce que** l'au moins un levier / l'au moins une manette (1) est composé(e) d'une matière plastique.

11. Poignée chirurgicale d'un instrument chirurgical selon l'une quelconque des revendications précédentes, **caractérisée en ce que** l'au moins un levier / l'au moins une manette (1) est en plusieurs parties.

12. Poignée chirurgicale d'un instrument chirurgical selon l'une quelconque des revendications précédentes, **caractérisée en ce que** l'au moins un levier / l'au moins une manette (1) est configuré(e) de manière à présenter une forme de coque.

13. Poignée chirurgicale d'un instrument chirurgical selon l'une quelconque des revendications précédentes, **caractérisée en ce que** l'au moins un levier / l'au moins une manette (1) entoure le boîtier de poignée (2, 2a) au moins par endroits à la manière d'un manchon, dans la direction périphérique de l'axe de poignée (8).

14. Instrument chirurgical à tige tubulaire constitué d'un outil à tige tubulaire, d'une tige d'instrument, au niveau de l'extrémité distale de laquelle l'outil à tige tubulaire est disposé, et d'une poignée chirurgicale selon l'une quelconque des revendications précédentes, laquelle est disposée ou peut être disposée au niveau de l'extrémité proximale de la tige d'instrument.
